# EUROPEAN PATENT APPLICATION

(11) **EP 4 714 711 A1**
(43) Date of publication of application: **25.03.2026**
(21) Application number: 24201093.2
(22) Date of filing: 18.09.2024
(51) Int. Cl.: B60K 35/00, A61B 5/16, A61B 5/18, B60K 35/21, B60K 35/22, B60K 35/25, B60K 35/26, G06V 20/59, B60W 40/08

(54) **METHOD AND SYSTEM FOR GENERATING A PERSONALIZED EXPERIENCE FOR AN OCCUPANT OF A VEHICLE**

(71) Applicant: Harman Becker Automotive Systems GmbH, 76307 Karlsbad (DE)
(72) Inventor: HARIHARAKRISHNAN, Anilkumar, 76307 Karlsbad (DE); IMRAN, Muneeb, 76307 Karlsbad (DE); JULIANO, Julia, 76307 Karlsbad (DE); GLINKA, Marta, 76307 Karlsbad (DE)
(74) Representative: Westphal, Mussgnug & Partner, Patentanwälte mbB

(57) **Abstract**

A method, system and computer program for generating a personalized experience for an occupant of a vehicle includes: capturing first occupant related data and processing the first occupant related data to estimate state and arousal of the occupant; capturing second occupant related data and processing the second occupant related data to estimate emotions and valence of the occupant; estimating a personality trait based on the state and arousal of the occupant, and the emotions and valence of the occupant; and generating the personalized experience for the occupant based on the personality trait of the occupant and at least one of the state and arousal of the occupant and the emotions and valence of the occupant, the personalized experience for the occupant comprising at least one of an acoustic presentation, a visual presentation, an olfactory presentation and a tactile presentation

## Description

### BACKGROUND

### 1. Technical Field

The disclosure relates to a method and system for generating a personalized experience for an occupant of a vehicle.

### 2. Related Art

It is becoming increasingly common to automatically detect a person's mental state, such as arousal, valence, concentration, irritation, attention, etc. For example, driving a motor vehicle while in a poor state of mind is a significant cause of driver error and potentially preventable traffic accidents. Vehicle systems that help alert drivers to their state and take action in such an event may reduce the number of traffic accidents or otherwise mitigate the disturbance caused by driver distraction. However, known systems and methods for assessing the mental state of a person may be inaccurate, resulting in an inappropriate vehicle response. Therefore, a more accurate assessment is desirable so as to produce an appropriate response.

### SUMMARY

A method for generating a personalized experience for an occupant of a vehicle includes: capturing first occupant related data and processing the first occupant related data to estimate state and arousal of the occupant; capturing second occupant related data and processing the second occupant related data to estimate emotions and valence of the occupant; and estimating a personality trait based on the state and arousal of the occupant, and the emotions and valence of the occupant. The method further comprises generating the personalized experience for the occupant based on the personality trait of the occupant and at least one of the state and arousal of the occupant and the emotions and valence of the occupant, the personalized experience for the occupant comprising at least one of an acoustic presentation, a visual presentation, an olfactory presentation and a tactile presentation

A system for generating a personalized experience for an occupant of a vehicle includes: at least one sensor disposed in a cabin of the vehicle and configured to capture first and second occupant related data; at least one actuating element disposed in the cabin of the vehicle; a memory configured to store instructions; and one or more processors operatively coupled to the at least one sensor, the at least one actuating element, and the memory. The one or more processors are configured, when executing the instructions stored in the memory, to receive the first occupant related data and process the first occupant related data to estimate state and arousal of the occupant; receive the second occupant related data and process the second occupant related data to estimate emotions and valence of the occupant; estimate a personality trait based on the state and arousal of the occupant, and the emotions and valence of the occupant; and generate the personalized experience for the occupant based on the personality trait of the occupant and at least one of the state and arousal of the occupant and the emotions and valence of the occupant, the personalized experience for the occupant comprising at least one of an acoustic presentation, a visual presentation, an olfactory presentation and a tactile presentation.

A computer program product embodied in a non-transitory computer readable medium for generating a personalized experience for an occupant of a vehicle, the computer program product comprising code which causes one or more processors to perform operations of: receiving the first occupant related data and processing the first occupant related data to estimate state and arousal of the occupant; receiving the second occupant related data and processing the second occupant related data to estimate emotions and valence of the occupant; estimating a personality trait based on the state and arousal of the occupant, and the emotions and valence of the occupant; and generating the personalized experience for the occupant based on the personality trait of the occupant and at least one of the state and arousal of the occupant and the emotions and valence of the occupant, the personalized experience for the occupant comprising at least one of an acoustic presentation, a visual presentation, an olfactory presentation and a tactile presentation.

Other systems, methods, features and advantages will be, or will become, apparent to one with skill in the art upon examination of the following detailed description and appended figures. It is intended that all such additional systems, methods, features and advantages be included within this description, be within the scope of the invention, and be protected by the following claims.

### BRIEF DESCRIPTION OF THE DRAWINGS

The system may be better understood with reference to the following drawings and description. The components in the figures are not necessarily to scale, emphasis instead being placed upon illustrating the principles of the invention.
FIG. 1 is a schematic diagram illustrating the relationship between personality, emotions /valence and state/arousal.
FIG. 2 is a block diagram illustrating signal flow paths and processes involved in an example method for generating a personalized experience for an occupant of a vehicle.
FIG. 3 is a schematic diagram illustrating a valence and arousal circumplex.
FIG. 4 is a table linking a type of presentation based on at least one of a detected arousal and valence state to an adjustment of the presentation dependent on a detected personality trait.

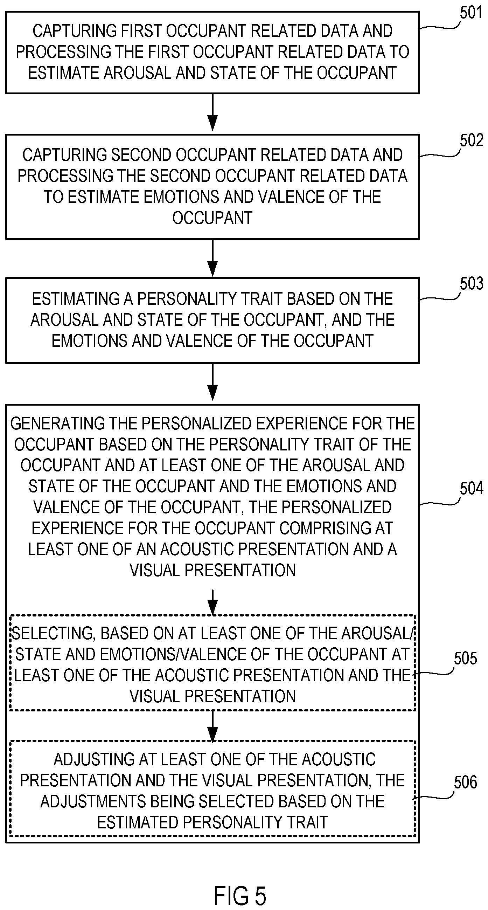

FIG. 5 is a flow chart illustrating a basic method for generating a personalized experience for an occupant of a vehicle.

### DETAILED DESCRIPTION

In modern psychology, arousal is the physiological and psychological state of being awoken or of sense organs stimulated to a point of perception. It involves activation of the ascending reticular activating system (ARAS) in the brain, which mediates wakefulness, the autonomic nervous system, and the endocrine system, leading to increased heart rate and blood pressure and a condition of sensory alertness, desire, mobility, and reactivity. Arousal is mediated by several neural systems. Wakefulness is regulated by the ARAS, which is composed of projections from five major neurotransmitter systems that originate in the brainstem and form connections extending throughout the cortex; activity within the ARAS is regulated by neurons that release the neurotransmitters norepinephrine, acetylcholine, dopamine, serotonin and histamine. Activation of these neurons produces an increase in cortical activity and subsequently alertness.

Vigilance, also termed sustained concentration, is commonly taken to mean the state of being alertly watchful and is defined as the ability to maintain concentrated attention over prolonged periods of time. During these periods, the person attempts to detect the appearance of a particular target stimulus. The individual watches for a signal stimulus that may occur at an unknown time. Reductions in arousal generally correspond to reductions in vigilance. Arousal is defined as a component of vigilance, although a reduction is not, as one may believe, the sole cause of vigilance decrement. Vigilance decrement is defined as "deterioration in the ability to remain vigilant for critical signals with time, as indicated by a decline in the rate of the correct detection of signals". Vigilance decrement is most commonly associated with monitoring to detect a weak target signal. A deterioration of detection performance is less likely to occur in cases in which the target signal exhibits a high saliency. For example, a radar operator would be unlikely to miss a rare target at the end of a watch if it were a large bright flashing signal, but might miss a small dim signal.

Emotions are usually caused by neurophysiological changes, variously associated with thoughts, feelings, behavioral responses, and a degree of pleasure or displeasure. There is no scientific consensus on the definition of emotions. Emotions are often intertwined with mood, temperament, disposition, or creativity. From a mechanistic perspective, emotions can be defined as "a positive or negative experience that is associated with a particular pattern of physiological activity." Emotions are complex, involving multiple different components, such as subjective experience, cognitive processes, expressive behavior, psychophysiological changes, and instrumental behavior.

Valence, also known as hedonic tone, is a characteristic of emotions that determines their emotional affect (intrinsic appeal or repulsion). Positive valence corresponds to the "goodness" or attractiveness of an object, event, or situation, making it appealing or desirable. Conversely, negative valence relates to "badness" or averseness, rendering something unappealing or undesirable. This concept is not only used to describe the intrinsic qualities of objects and events but also categorizes emotions based on their inherent attractiveness or averseness.

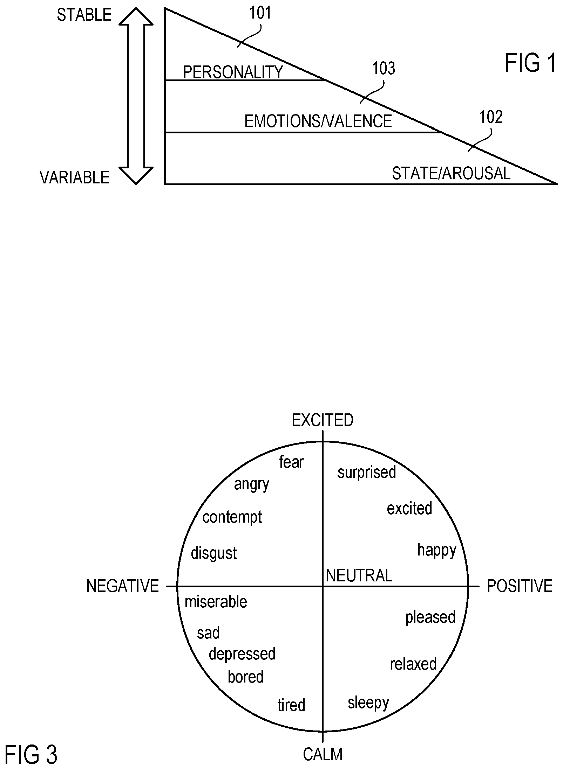

Personality is the characteristic set of an individual's behavioral and emotional patterns that evolve from biological and environmental factors. There are five basic dimensions of personality among others: extraversion, agreeableness, openness, conscientiousness, and neuroticism. Personality is thought to be more stable than emotion and a great deal more stable than arousal, as depicted in FIG. 1, in which personality 101 is shown on top of a pyramid, with state/arousal (state and arousal) 102 as the most variable condition at the bottom of the pyramid and emotions/valence (emotions and valence) 103 in between. These personality factors impact the autonomic nervous system and can thus affect acute bodily responses, such as heart rate and blood pressure, as well as physiological signals such as electroencephalograms (EEG), electrocardiograms (ECG), electromyograms (EMG), explaining, in part, individual differences in emotion and arousal (detection). For example, neuroticism is associated with higher arousal, especially for negative valence stimuli. Moreover, effectively returning to an optimal arousal state can also depend on personality factors (intervention).

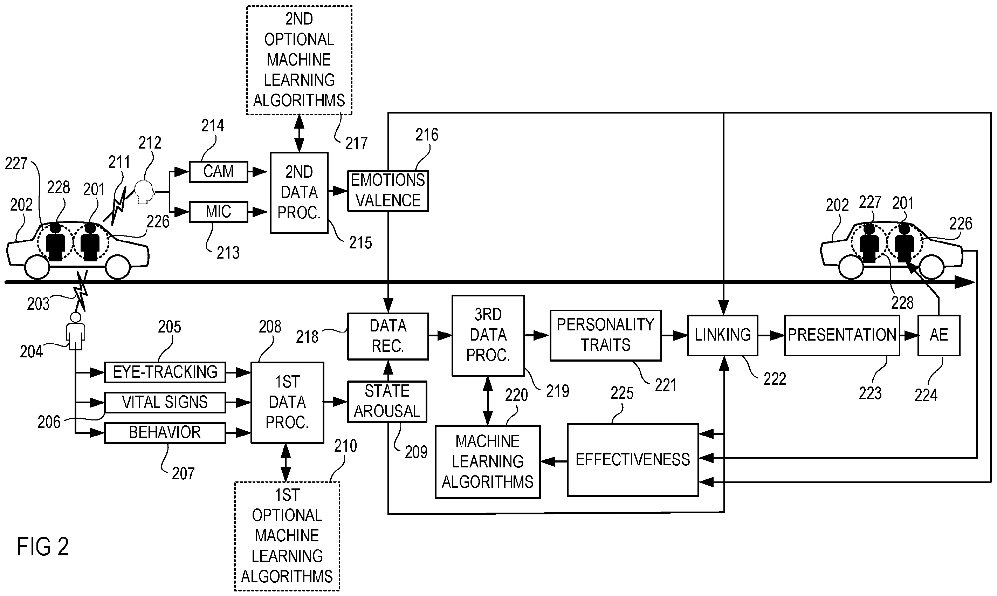

FIG. 2 shows signal flow paths and procedures involved in an example method for generating a personalized experience for an occupant 201 of a vehicle 202. The occupant 201 may be any person in any position in any vehicle. In the example method shown in and described in connection with FIG. 2, it is assumed that the occupant is the driver of the vehicle 202 and the vehicle 202 is a car. Alternatively, the vehicle 202 may be a plane, ship, van, bus, train etc. Alternatively, the occupant 201 may be another passenger of the car or a passenger of the plane, ship, van, bus, or train, or an operator of these vehicles.

Initially, the occupant receives one or more external stimuli, e.g., stimuli 203 and 211, which may include, for example, a tone or light generated inside the vehicle 202, a text message on a screen inside the vehicle 202, a positive or negative acceleration of the vehicle, etc. These stimuli 203 and 211 have in common that they are generated (e.g., text, tone light) or are detectable by the vehicle 202 (e.g., acceleration or temperature), so that the stimuli that occur can be identified. Certain stimuli 203 provoke certain physiological responses 204 such as eye movements, changes in at least one of the vital signs (e.g., at least one of EEG, ECG, EMG, blood pressure, and heartrate), and movement of the body and the head.

The physiological responses 204 are detectable and can thus be assigned individually to the detected stimuli 203. One or more specific sensors such as, for example, at least one of a camera, microphone, eye tracker, electroencephalography, electrocardiography, heartrate monitor, blood pressure monitor, and accelerometer may be employed to measure the physiological responses 204. In the example method shown in FIG. 2, an eye-tracker 205, a vital signs detector 206 for measuring multiple vital signs, and at least one camera 207 for capturing the occupant's behavior including occupant's body and head movements are used as sensing technology. The results of the measurements of the physiological responses 204 are provided as first data sets to a first data processing procedure 208 which processes the first data sets to extract therefrom specific parameters that describe the state/arousal 209 of the person under investigation, e.g.., the occupant 201. The first data processing procedure 208 may use one or more first optional machine learning algorithms 210 to compute the specific parameters which describe the state/arousal 209 of the occupant 201.

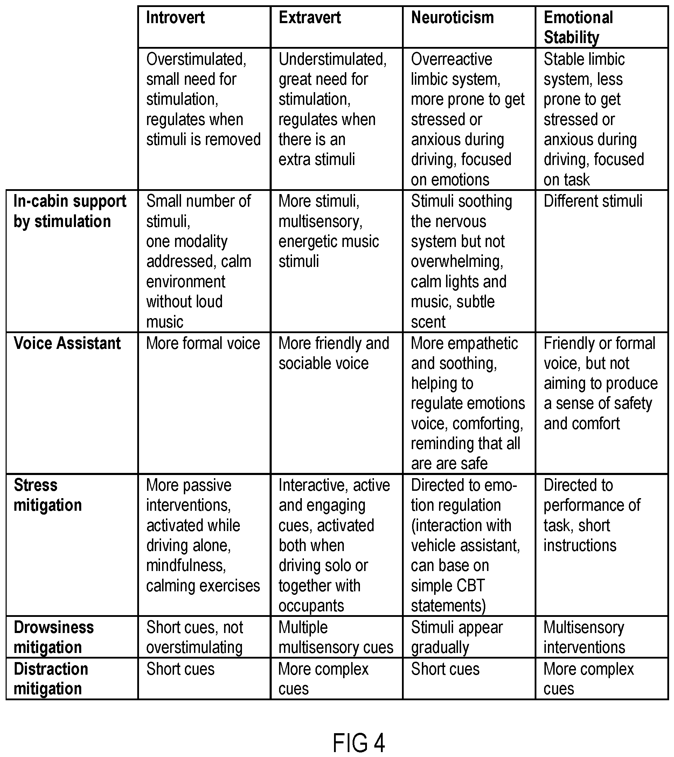

The stimuli 211 also evoke emotional reactions 212 in the passenger 201 such as changes in at least one of the voice pitch and facial expression, which are monitored by at least one of a microphone (MIC) 213 and a camera (CAM) 214 to provide second data sets. A second data processing procedure 215 processes the second data sets to extract therefrom specific parameters that describe the emotions/valence 216 of the occupant 201. The second data processing procedure 215 may use second optional machine learning algorithms 217. The specific parameters that describe the state/arousal 209 of the occupant 201 and the specific parameters that describe the emotions/valence 216 of the occupant 201 are continuously stored in a data recording unit 218 (e.g., a memory, register etc.). The time period for how long the parameters that describe the state/arousal 209 are stored may be (significantly) longer than the time period for how long the parameters that describe the emotions/valence 216 are stored. A third data processing procedure 219 using machine learning algorithms 220 computes personality traits 221 from the parameters stored in the data recording unit 218. The personality traits 221 are linked with the emotions/valence 216 and the state/arousal 209 in a linking procedure 222 to select or adjust a presentation 223 for the occupant 201. Examples for adjustments of selected presentations linked to the personality and at least one of the arousal/state of the occupant 201 and the emotions/valence of the occupant 201 are described below in connection with FIG. 4.

At least two of the first data processing procedure 208, the second data processing procedure 215, the third data processing procedure 215, the machine learning algorithms 220, the first optional machine learning algorithms 210, and the second optional machine learning algorithms 217 may run on the same processor(s) or may run on at least one separate processor. The first optional machine learning algorithms 210 and the second optional machine learning algorithms 217 may exchange data for optimization of the algorithms (not shown in FIG.2). The at least one microphone 213 and at least one camera 214 may be co-used by the sensing technology employed in connection with estimating the state/arousal 209 and the emotions/valence 216.

The presentation 223 controls one or more actuating elements (AE) 224 in the vehicle 202. The one or more actuating elements 224 may include, for example, at least one of a loudspeaker, display, light-emitting diode, tone generator, speech generator, text generator, electro-mechanical actuator, voice synthesis processor, and text generator to provide a personalized experience to the occupant 201. An effectiveness 225 of the adjustments, i.e., the effectiveness of the interventions affecting the emotions/valence 216, state/arousal 209 and the driving behavior, may be monitored by the machine learning algorithm 220. The effectiveness 225 may be based on emotion/valence to a lesser extent than on state/arousal. The adjustments may be optimized based on the detected effectiveness 220. The method described above with regard to occupant 201 in a zone 226 can be executed zonewise, i.e., separately for separate zones as, for example, for at least one other occupant 227 in a zone 228.

The personalized experience of the occupant 201 evoked by the presentation 223 may include at least one of acoustic presentation, visual presentation, olfactory presentation and tactile presentation. The acoustic presentation may include at least one of speech, music, warning tones, and noise, wherein adjusting the acoustic presentation may include changing at least one of the tone, volume, timely structure, frequency range, repetition rate, timbre and pitch of a voice, speech impediment, and wording. The visual presentation may include at least one of controlling lamps, and providing presentations on a display including graphics, pictures, videos and text, wherein adjusting the visual presentation comprises changing at least one of color, brightness, pulsing, fonts type, optical structure, size, text and repetition rate of the visual presentation. The olfactory presentation may include spraying of scents in the vehicle cabin. The tactile treatment may include shaking or vibrating, e.g., of the seat, steering wheel etc.

FIG. 3 depicts a valence and arousal circumplex as described by Toisoul, A., Kossaifi, J., Bulat, A., Tzimiropoulos, G., & Pantic, M., (2021), Estimation of continuous valence and arousal levels from faces in naturalistic conditions. Nature Machine Intelligence, 3(1), 42-50. The various valence and arousal states are distributed on a circular line around a circle center marked "neutral". The circular line is divided into four circular segments by a coordinate system with the center point "neutral" as the origin, whereby the x-axis represents the valence state and the y-axis the state of arousal. The valence (x-axis), which indicates how negative or positive the emotional state is, ranges accordingly from a negative state through neutral to a positive state. The arousal (y-axis), which indicates how calming or exciting the emotional state is, ranges accordingly from a state of "calm" via "neutral" to an excited state.

Between the negative valence state and the excited arousal state, there are the following combined valence and arousal states along this segment of the circle line: disgusted (disgust), contemptuous (contempt), angry, and fearful (fear). Between the excited arousal state and the positive valence state, there are the following combined valence and arousal states along this segment of the circle line: surprised, excited and happy. Between the positive valence state and the calm arousal state, there are the following combined valence and arousal states along this segment of the circle line: pleased, relaxed, sleepy. Between the calm arousal state and the negative valence state, there are the following combined valence and arousal states along this segment of the circle line: tired, bored, depressed, sad, and miserable.

Based on these findings, Toisoul et al., further describe a method for the estimation of continuous valence and arousal levels from faces in naturalistic conditions, in which valence and arousal is continuously estimated from images of a facial display recorded in naturalistic conditions using a deep neural network architecture that jointly performs facial alignment and correctly predicts both discrete and continuous emotion labels in a single pass based on a simplified pipeline of emotion recognition from facial imagery. This allows important facial features around facial points to be used to build an attention mechanism, thus improving estimation performance. It also facilitates implementation as a single-step method with a lightweight model that runs in real time.

The method presented by Toisoul et al. may be applied in the system and method described herein to detect the valence and arousal state. Other, at least partly applicable, methods for detecting the valence and arousal state are described, for example, in US 10,453,453 B2, US 10,796,176 B2, US 10,922,567 B2, US 11,067,405 B2, US 10,627,817 B2, US 10,592,757 B2, US 10,867,218 B2, and US 2021/0001862 A1. Personality traits may be determined in a manner described, for example, in US 9,576,571 B2, US 9,805,128 B2, US 10,049,103 B2, US 10,311,869 B2, and US 2019/0206546 A1. Unlike valence and arousal states, which can change from moment to moment, personality traits tend to be stable. Therefore the data collected in order to assess personality can be collected over a longer period of time.

FIG. 4 shows an example table linking different types of presentations (lines of the table) selected based on a detected state/arousal or emotions/valence or both to an adjustment of the presentations based on detected personality traits (columns of the table) to provide an adjusted occupant experience. For example, a certain state/arousal or certain emotions/valence or both cause certain responses such as in-cabin support by stimulation (e.g., scents, music, and lights), voice assistance, stress mitigation, drowsiness mitigation, and distraction mitigation. Possible personality traits may be: introversion, extraversion, neuroticism, and emotional stability. Introversion can sometimes include being more susceptible to overstimulation, or having little need for stimulation. Introversion will be regulated when the stimuli are removed. Extraversion can sometimes include being under-stimulated, or having a need for stimulation. Extraversion will be regulated when there is an extra stimulus. Neuroticism can sometimes include an over-reactive limbic system which can result in a strong tendency to become stressed or anxious while driving and focus on emotions. Emotional stability can sometimes include having a stable limbic system which can result in a weaker tendency (e.g., a less frequent behavior) to become stressed or anxious while driving and a clear focus on the task.

When a desire or need for an in-cabin support by stimulation is detected due to the state/arousal analysis and/or emotions/valence analysis, a small number of stimuli may be chosen, only one modality may be addressed, and a calm environment without loud music may be selected for introvert persons. However, for extravert persons, more stimuli and multisensory and multisensory, energetic music may be chosen. For persons with neurotic personality, stimuli that are soothing but not overwhelming for the nervous system may be selected in connection with calm lights and music, with maybe a subtle scent. Emotionally stable persons could receive different stimuli.

When a desire or need for a voice assistant is detected due to the state/arousal analysis and/or emotions/valence analysis, a more formal voice may be chosen for introvert persons, and a more friendly and sociable voice for extravert persons. A more empathetic and soothing voice that helps to regulate emotions, is comforting and reminds the passenger that they are safe may be selected for persons with a neurotic personality. A friendly or formal voice that does not aim to produce a sense of safety and comfort may be used in connection with emotionally stable persons.

When a desire or need for stress mitigation is detected due to the state/arousal analysis and/or emotions/valence analysis, introvert persons may receive more passive interventions, e.g., activation while driving alone, measures to increase mindfulness, and calming exercises. Extravert persons may receive interactive, active and engaging cues that are activated both when driving solo or together with other occupants. Measures directed to emotion regulation such as interaction with a vehicle assistant system, e.g., based on simple Cognitive Behavioral Therapy (CBT) statements, may be presented to persons with a neurotic personality. Emotionally stable persons may receive short instructions that are directed to performance of the task at hand.

When a desire or need for distraction mitigation is detected due to the state/arousal analysis and/or emotions/valence analysis, introvert persons may receive short, not overstimulating cues. Extravert persons receive multiple multisensory cues. Persons with a neurotic personality receive stimuli that appear gradually. Emotionally stable persons receive multisensory interventions. When a desire or need for stress mitigation is detected due to the state/arousal analysis and/or emotions/valence analysis, introvert persons and persons with neurotic personality receive short cues, while extravert persons and emotionally stable persons receive more complex cues.

A method for generating a personalized experience for an occupant of a vehicle includes capturing first occupant related data and processing the first occupant related data to estimate state and arousal of the occupant (procedure 501); capturing second occupant related data and processing the second occupant related data to estimate emotions and valence of the occupant (procedure 502); estimating a personality trait based on the state and arousal of the occupant, and the emotions and valence of the occupant (procedure 503); and generating the personalized experience for the occupant based on the personality trait of the occupant and at least one of the state and arousal of the occupant and the emotions and valence of the occupant, the personalized experience for the occupant comprising at least one of an acoustic presentation and a visual presentation (procedure 504). Procedure 503 may include selecting, based on the at least one of the state and arousal of the occupant and the emotions and valence of the occupant, at least one of the acoustic presentation and the visual presentation (sub-procedure 505), and adjusting at least one of the acoustic presentation and the visual presentation, the adjustments being selected based on the estimated personality trait (sub-procedure 506).

The systems and methods described above provide personalized experiences to occupants through the combination of personality with arousal and valence state detection. By considering the occupant's personality traits, the system can add additional personalization benefits in the form of experiences that are tailored to match personality characteristics. For interventions that return occupants to an optimal arousal state, the integration of personality traits increases their effectiveness. Personalizing experiences also increases occupant acceptance and satisfaction of the system and method.

Common vehicle systems that detect occupant's arousal and valence state and offer interventions do not consider personality factors. This results in misclassification and ineffective personalization, which can result in user dissatisfaction. It has been found that vehicle systems that provide interventions and experiences to occupants that are solely based on instantaneous state and arousal are more effective if they are tailored to the individual's personality. Without knowledge of the personality from interventions and experiences, the system risks being ineffective and annoying. The systems and methods described above incorporate personality trait information with system interventions and experiences.

The method described above may be encoded in a computer-readable medium such as a CD ROM, disk, flash memory, RAM or ROM, an electromagnetic signal, or other machine-readable medium as instructions for execution by a processor. Alternatively or additionally, any type of logic may be utilized and may be implemented as analog or digital logic using hardware, such as one or more integrated circuits (including amplifiers, adders, delays, and filters), or one or more processors executing amplification, adding, delaying, and filtering instructions; or in software in an application programming interface (API) or in a Dynamic Link Library (DLL), functions available in a shared memory or defined as local or remote procedure calls; or as a combination of hardware and software.

The method may be implemented by software and/or firmware stored on or in a computer-readable medium, machine-readable medium, propagated-signal medium, and/or signal-bearing medium. The media may comprise any device that contains, stores, communicates, propagates, or transports executable instructions for use by or in connection with an instruction executable system, apparatus, or device. The machine-readable medium may selectively be, but is not limited to, an electronic, magnetic, optical, electromagnetic, or infrared signal or a semiconductor system, apparatus, device, or propagation medium. A non-exhaustive list of examples of a machine-readable medium includes: a magnetic or optical disk, a volatile memory such as a Random Access Memory "RAM," a Read-Only Memory "ROM," an Erasable Programmable Read-Only Memory (i.e., EPROM) or Flash memory, or an optical fiber. A machine-readable medium may also include a tangible medium upon which executable instructions are printed, as the logic may be electronically stored as an image or in another format (e.g., through an optical scan), then compiled, and/or interpreted or otherwise processed. The processed medium may then be stored in a computer and/or machine memory.

The systems may include additional or different logic and may be implemented in many different ways. A processor or controller may be implemented as a microprocessor, microcontroller, application specific integrated circuit (ASIC), discrete logic, or a combination of other types of circuits or logic. Similarly, memories may be DRAM, SRAM, Flash, or other types of memory. Parameters (e.g., conditions and thresholds) and other data structures may be separately stored and managed, may be incorporated into a single memory or database, or may be logically and physically organized in many different ways. Programs and instruction sets may be parts of a single program, separate programs, or distributed across several memories and processors.

The description of embodiments has been presented for purposes of illustration and description. Suitable modifications and variations to the embodiments may be performed in light of the above description or may be acquired from practicing the methods. For example, unless otherwise noted, one or more of the described methods may be performed by a suitable device and/or combination of devices. The described methods and associated actions may also be performed in various orders in addition to the order described in this application, in parallel, and/or simultaneously. The described systems are exemplary in nature, and may include additional elements and/or omit elements.

As used in this application, an element or step recited in the singular and proceeded with the word "a" or "an" should be understood as not excluding plural of said elements or steps, unless such exclusion is stated. Furthermore, references to "one embodiment" or "one example" of the present disclosure are not intended to be interpreted as excluding the existence of additional embodiments that also incorporate the recited features. The terms "first," "second," and "third," etc. are used merely as labels, and are not intended to impose numerical requirements or a particular positional order on their obj ects.

While various embodiments of the invention have been described, it will be apparent to those of ordinary skilled in the art that many more embodiments and implementations are possible within the scope of the invention. In particular, the skilled person will recognize the interchangeability of various features from different embodiments. Although these techniques and systems have been disclosed in the context of certain embodiments and examples, it will be understood that these techniques and systems may be extended beyond the specifically disclosed embodiments to other embodiments and/or uses and obvious modifications thereof.

## Claims

1. A method for generating a personalized experience for an occupant of a vehicle, the method comprising:
capturing first occupant related data and processing the first occupant related data to estimate state and arousal of the occupant;
capturing second occupant related data and processing the second occupant related data to estimate emotions and valence of the occupant;
estimating a personality trait based on the state and arousal of the occupant, and the emotions and valence of the occupant; and
generating the personalized experience for the occupant based on the personality trait of the occupant and at least one of the state and arousal of the occupant and the emotions and valence of the occupant, the personalized experience for the occupant comprising at least one of an acoustic presentation, a visual presentation, an olfactory presentation and a tactile presentation.

2. The method of claim 1, wherein the first occupant related data and the second occupant related data are derived using at least one of still pictures, videos, voice recordings, eye tracking, and vital signs of the occupant.

3. The method of claim 1 or 2, wherein:
the personality trait comprises multiple categories of personality;
the categories include at least one of introvert, extravert, neuroticism, emotional stability; and
each category corresponds to a specific adjustment setting of the acoustic presentation, visual presentation, olfactory presentation and tactile presentation.

4. The method of any of the preceding claims, wherein the acoustic presentation comprises at least one of speech, music, warning tones, and noise.

5. The method of claim 4, wherein adjusting the acoustic presentation comprises changing at least one of the tone, volume, timely structure, frequency range, repetition rate, timbre and pitch of a voice, speech impediment, and wording.

6. The method of any of the preceding claims, wherein the visual presentation comprises at least one of controlling lamps, and providing graphics, pictures, videos and text on a display.

7. The method of claim 6, wherein adjusting the visual presentation comprises changing at least one of color, brightness, pulsing, fonts type, optical structure, size, and repetition rate.

8. The method of any of the preceding claims, further comprising monitoring the effectiveness of the adjustments of the acoustic presentation or visual presentation or both, and optimizing the adjustments of at least one of the acoustic presentation, visual presentation, olfactory presentation and tactile presentation based on the effectiveness.

9. The method of any of the preceding claims, wherein the personality trait is estimated using a machine learning algorithm.

10. The method of any of the preceding claims, wherein the first and second occupant related data comprise external stimuli and their physiological responses.

11. The method of any of the preceding claims, further comprising generating another personalized experience for another occupant of the vehicle in the same manner as for the one occupant, the personalized experiences are restricted to zones around the occupants.

12. A system for generating a personalized experience for an occupant of a vehicle comprising at least one sensor disposed in a cabin of the vehicle and configured to capture first and second occupant related data, at least one actuating element disposed in the cabin of the vehicle, a memory configured to store instructions, and one or more processors operatively coupled to the at least one sensor, the at least one actuating element, and the memory; wherein the one or more processors, when executing the instructions stored in the memory, are configured to:
receive the first occupant related data and process the first occupant related data to estimate state and arousal of the occupant;
receive the second occupant related data and process the second occupant related data to estimate emotions and valence of the occupant;
estimate a personality trait based on the state and arousal of the occupant, and the emotions and valence of the occupant; and
generate the personalized experience for the occupant based on the personality trait of the occupant and at least one of the state and arousal of the occupant and the emotions and valence of the occupant, the personalized experience for the occupant comprising at least one of an acoustic presentation, a visual presentation, an olfactory presentation and a tactile presentation.

13. The system of claim 12, wherein the at least one sensor comprises at least one of a camera, microphone, eye tracker, electroencephalography, electrocardiography, heartrate monitoring, blood pressure monitoring, and accelerometer.

14. The system of claim 12 or 13, wherein the at least one actuating element comprises at least one of a loudspeaker, display, light-emitting diode, tone generator, speech generator, text generator, and electro-mechanical actuator.

15. A computer program product embodied in a non transitory computer readable medium for generating a personalized experience for an occupant of a vehicle, the computer program product comprising code which causes one or more processors to perform operations of:
receiving the first occupant related data and processing the first occupant related data to estimate state and arousal of the occupant;
receiving the second occupant related data and processing the second occupant related data to estimate emotions and valence of the occupant;
estimating a personality trait based on the state and arousal of the occupant, and the emotions and valence of the occupant; and
generating the personalized experience for the occupant based on the personality trait of the occupant and at least one of the state and arousal of the occupant and the emotions and valence of the occupant, the personalized experience for the occupant comprising at least one of an acoustic presentation, a visual presentation, an olfactory presentation and a tactile presentation.
